# EUROPEAN PATENT APPLICATION

(11) **EP 4 350 489 A1**
(43) Date of publication of application: **10.04.2024**
(21) Application number: 22200186.9
(22) Date of filing: 07.10.2022
(51) Int. Cl.: G06F 3/03, A61B 5/11, A61B 5/22, A61B 5/00, B43K 29/08, G06F 3/0346, G06F 3/0354

(54) **WRITING INSTRUMENT AND METHOD THEREFOR**

(71) Applicant: BIC Violex Single Member S.A., 145 69 Anoixi (GR)
(72) Inventor: Chrysanthakopoulos, Nikolaos, 145 69 Anoixi (GR); Antonakis, Ion - Ioannis, 145 69 Anoixi (GR); Karakalas, Anargyros, 145 69 Anoixi (GR); Tsagkrasoulis, Dimosthenis, 145 69 Anoixi (GR)
(74) Representative: Peterreins Schley

(57) **Abstract**

The present disclosure relates to a computer-implemented method for monitoring handwriting and/or sketching changes of a writing instrument's user, comprising:
- monitoring a handwriting and/or sketching trajectory and motion parameters of the user during a writing and/or sketching session with the writing instrument (10) by reading data from the sensors of the writing instrument (10);
- evaluating letter and word segmentation and identification data obtained from the monitoring;
- quantifying size and distance of letters and/or sketch components and parameters of the evaluated data;
- storing monitored data and/or quantified data as historical data;
- comparing the quantified data to at least one of data from the actual writing and/or sketching session, historical data from the same user, and reference data from a group of users; and
- providing an indication in case of an abnormal result of the comparing.

## Description

### Technical Field

The present disclosure relates to a method for monitoring handwriting and/or sketching changes of a writing instrument's user and a writing instrument for monitoring handwriting and/or sketching changes of its user.

### Background

Alzheimer's Disease (AD) is a form of dementia, a neurodegenerative disease of the brain. The disease process is associated with amyloid plaques, neurofibrillary tangles, and loss of neuronal connections in the brain. Unfortunately, the cause of Alzheimer's disease is poorly understood. There are many environmental and genetic risk factors associated with it. The strongest genetic risk factor is from an allele of APOE gene. Other risk factors are history of head injury, clinical depression, and high blood pressure.

Parkinson's disease (PD) is a long-term degenerative disorder of the central nervous system that mainly affects the motor system. The cause of PD is unknown, with both inherited and environmental factors believed to play a role. The symptoms are due to the death of cells in the substantia nigra, a region of the midbrain, leading to a dopamine deficit. The cause of this cell death is poorly understood but involves the build-up of misfolded proteins into Lewy bodies in the neurons.

Micrographia is an acquired disorder that features abnormally small, cramped handwriting or the progression to progressively smaller handwriting. It is commonly associated with neurodegenerative disorders of the basal ganglia, such as in Parkinson's disease, but it has also been ascribed to subcortical focal lesions. Micrographia is also seen in patients with Wilson's disease, Obsessive Compulsive Disorder, Metamorphopsia, or with isolated focal lesions of the midbrain or basal ganglia.

People with Parkinson's disease tend to find their handwriting gets smaller, even though they don't intend to write smaller. In Parkinson's, the words you write may be closer together on the page (even crowded together so that they're difficult to read), and your letter sizes may be smaller, too. Finally, your writing may tilt upward to the right on the page. All these are signs of micrographia.

The micrographic signature is a reduction in size but not a change of morphology or style. Individuals with micrographia may show a reduction of letter size over time within a trial.

However, the letter size may increase significantly when either the individuals are given visual targets or constant auditory reminders. The letter amplitude may also increase when the individuals are required to write between parallel lines.

The Mini-Cog is a 3-minute test that can assist in the detection of cognitive impairment in older adults. It can be used effectively after brief training in both healthcare and community settings. It consists of two components, a 3-item recall test for memory and a simply scored clock drawing test.

For the clock drawing test, the patient is asked to draw an analogue clock and put all the numbers in the correct position of the circle and then draw the clock hands at a specific hour and minute. A doctor evaluates the design and rates the cognitive status of the patient. A normal clock must include all numbers (1-12), each only once, in the correct order and direction (clockwise). An abnormal clock may have one of the two hands missing, or it may not point to the requested times, or have one or more clock times missing.

There are other sketches alternative to clock drawing, used for diagnostics of MCI or AD proposed in scientific literature. For example, the sketches of a turtle, strawberry, train, and envelope are items that best discriminated between groups of healthy and not healthy persons. Other designs tested are infinity loops and wire cubes.

### Summary

In a first general aspect, the present disclosure relates to a computer-implemented method for monitoring handwriting and/or sketching changes of a writing instrument's user, comprising:
- monitoring a handwriting and/or sketching trajectory and motion parameters of the user during a writing and/or sketching session with the writing instrument by reading data from the sensors of the writing instrument;
- evaluating letter and word segmentation and identification data obtained from the monitoring;
- quantifying size and distance of letters and/or sketch components and parameters of the evaluated data;
- storing monitored data and/or quantified data as historical data;
- comparing the quantified data to at least one of data from the actual writing and/or sketching session, historical data from the same user, and reference data from a group of users; and
- providing an indication in case of an abnormal result of the comparing.

In embodiments, monitoring handwriting and/or sketching changes may include monitoring and/or identifying at least one of a writing force between the writing instrument's tip and a writing surface, motion and distance information including position, orientation, speed, and/or acceleration of the writing instrument, sequential visual data of the handwriting/sketching operation output, and a force distribution and/or gripping strength applied by the user's fingers on the writing instrument.

In embodiments, monitoring handwriting and/or sketching changes may include computing a time span of the writing instrument not in contact with the writing surface between two consecutive writing strokes from the monitored force.

In embodiments, writing instrument motion data may be derived from a magnetometer sensor and an inertial measurement unit, IMU, sensor of the writing instrument.

In embodiments, the method may comprise fusing writing instrument motion data through a data processing algorithm like e.g., a Kalman filter, wherein the writing instrument motion data are multidimensional, wherein the dimensions are at least one of position, orientation, speed, and acceleration.

In embodiments, the method may comprise sorting the fused writing instrument motion data into writing and non-writing parts by using data of a writing force between the writing instrument's tip and a writing surface.

In embodiments, the method may comprise digitizing handwriting input of the writing session; and correlating the digitized handwriting input with motion data of the writing instrument. In other words, the method may comprise digitizing physical laydown or digitizing handwriting and/or sketching output.

In embodiments, the method may comprise identifying at least one of size, spacing, and identification parameters of characters and/or shapes of the writing parts.

In embodiments, comparing may include comparing the current characters' and/or shapes' size and spacing parameters of the user to the historical characters and/or shapes size and spacing parameters of the same user and/or comparing current sketch properties of the user to a historical sketch and/or a reference sketch.

In embodiments, the method may comprise performing a check that all twelve numbers of a clock of a Cog test are written and that the order of writing is correct; providing straight lines between centers of the numbers' bounding boxes and comparing the resulting shape to a dodecagon; checking the existence of the two clock arrows; and extracting the length of the two clock arrows as the diagonal of the bounding box and the direction via distance of the arrow head to the closest bounding box corner.

In a second general aspect, the present disclosure relates to a writing instrument for monitoring handwriting and/or sketching changes of its user, comprising:
one or more sensors configured to monitor a handwriting and/or sketching trajectory and motion parameters of the user during a writing and/or sketching session with the writing instrument by reading sensors of the writing instrument;
a computer system configured to execute the computer-implemented method for monitoring handwriting and/or sketching changes of a writing instrument's user as described above;
a storage system configured to store monitored data and/or quantified data as historical data; and
a communication system configured to provide an indication based on the computer-implemented method.

In embodiments, the writing instrument may comprise at least one of:
a hand presence sensor configured to obtain a force distribution and/or a gripping strength applied by the user's fingers on the writing instrument;
a force tip sensor configured to obtain a writing force between the writing instrument's tip and a writing surface;
at least one motion sensor configured to obtain at least one of position, orientation, speed, and acceleration of the writing instrument;
a sensor, such as an optical sensor, configured to provide sequential visual data of the handwriting/sketching operation; and
a proximity sensor configured to obtain a hovering status of the writing instrument's tip above the writing surface.

The hovering or touching status of the pen's tip indicates whether the tip is on the paper which corresponds to a writing and/or sketching action or whether the tip is in the air which is indicative of non-writing and/or non-sketching time. Measurement of the hovering status can include measuring the distance between the tip and the writing surface.

In embodiments, the proximity sensor may be a time-of-flight sensor, wherein the hand presence sensor comprises a plurality of flexible pressure sensing pads, wherein the force tip sensor comprises a force and/or pressure sensor configured to measure a force and/or pressure of a tip of the writing instrument, wherein the optical sensor comprises an optical image stabilization mechanism and/or a digital image stabilization algorithm, and/or wherein the at least one motion sensor comprises at least one of a magnetometer, a gyroscope, an accelerometer, and an inertial measurement unit, IMU.

In embodiments, the writing instrument may comprise a digitizing system configured to digitize content of a writing and/or sketching session.

In embodiments, the writing instrument may comprise a user interface configured to receive input from a user and/or to provide information to the user such as the indication; and/or a wireless communication system configured to provide the indication via an external device having an interface.

According to examples of the present disclosure, abnormal handwriting and sketching properties associated with neurodegenerative diseases can be detected early with the use of a system comprised of a multifunctional pen equipped with several motion detection and optical sensors.

A writing instrument or smart pen may be capable of both being used as a conventional writing instrument, i.e., write or sketch on a writing surface, as well as capture data associated with the handwriting and/or sketching operation.

More specifically, a focus may be placed on evaluating data associated with micrographia handwriting disorder and Parkinson's disease, as well as established Sketching tests (like Cog test) and Alzheimer's disease.

Quantifiable metrics relevant to micrographia may include character size and inter-character/word distance. Analysis thus is based on letter/word segmentation and identification, followed by size and distance quantification. Comparison of these metrics for the same letters/words written early and later in a writing session may provide a robust indication of micrographia.

The above metrics can be extracted via the use of a smart pen equipped with a magnetometer, IMU and a pen tip force sensor for absolute position tracking.

The data from the first two sensor types may be fused to yield accurate position information of the pen tip, while the latter allows for distinction between pen in the paper (writing) vs pen in the air (non-writing) time.

The force sensor may be substituted by a Time-of-Flight sensor which can also be used to distinguish between writing vs. non-writing time.

In an alternate embodiment, the IMU can be substituted or complemented by an optical image sensor, which captures the writing parts as images. The positional coordinates from the magnetometer as well as the orientation of the writing instrument can then be used to combine the local optical images to a global picture through a combination of motion trajectory data and machine vision.

The device described may find additional use on the evaluation of patients with cognitive impairment. The Mini-Cog test includes the evaluation of a clock drawing, as described previously.

This task may be assessed via an automated system that provides a score on the performance of the writer. In more detail, the smart pen device yields a digitized image of the drawing, which is then analyzed using processing algorithms to identify and quantify the issues. The analysis requires segmentation and object detection of the written digits, as well as positional analysis of both the digits and the clock hands.

Steps of an example method of the present disclosure may be as follows:
- A user writes or sketches with his/her smart pen on a normal writing surface, as usually.
- Smart pen sensors detect handwriting/sketching trajectory and parameters (position, orientation, speed).
- Smart pen algorithms evaluate letter sizes, distance between characters and/or sketch components and parameters related to AD
- Smart pen algorithms alerts user whether micrographia or sketch inconsistencies are detected.

An example system of the present disclosure may provide the following features:
A position detecting sensor embedded on the pen body, like magnetometer, IMU
A hand presence detection sensor embedded on the pen body, like a gripping force sensor.

A writing action detection sensor embedded on the pen tip, like a tip force sensor.

A microcontroller in the smart pen for the control of the sensors, a memory unit for storing sensor data, a communication unit for transferring the logged data, a power source for the system, and at least one LED for indicating key operational statuses.

Particular examples of the first to second general aspects can be implemented so as to realize one or more of the following advantages.

In examples, the present disclosure enables identifying a chronic or degenerative disease in early stages which is very difficult in most cases.

In examples, the present disclosure enables to identify a chronic or neurodegenerative disease as the quality of handwriting is severely affected when medium to severe symptoms of a chronic or neurodegenerative disease are expressed.

In examples, the present disclosure overcomes issues where handwriting disorders are usually assessed qualitatively by physicians and cannot be easily identified and quantified.

In examples, the present disclosure overcomes issues where most diagnostic tests currently adopted for AD, PD and MCI require that the patient visits a dedicated facility where the patient might be subjected to complex and expensive tests performed by trained health-care professionals. This process is time-consuming, could be stressful for the patient as well as costly.

The present disclosure allows identifying a chronic or degenerative disease in early stages which is very difficult in most cases.

The present disclosure allows to detect and digitize handwriting input like e.g., letter(s), numeral(s) and word(s).

The present disclosure allows to detect and digitize sketching input.

The present disclosure allows to quantify handwritten letters size and distribution (distance between letters) with high accuracy and resolution during the use of a pen for trivial handwriting activities without any disturbance of the user.

The present disclosure allows to quantify sketching parameters with high accuracy during the use of a pen without any disturbance of the user.

The present disclosure allows to compare current user's handwriting and sketching input to historical data and normal database and find significant changes.

The present disclosure allows to evaluate micrographia and sketching inconsistencies related to cognitive dysfunction and neurodegenerative diseases.

The present disclosure allows to alert the user whether his handwriting and sketching properties are normal or abnormal.

Regarding smart pen handwriting and/or sketching data extraction sensors the implementation of smart, wearable sensors and systems in almost any device is possible due to the low power consumption, minimal obtrusiveness, and low weight. Several sensing technologies can be implemented independently or in tandem to capture the writing motor skills of a person while using a writing instrument such as a pen, pencil, marker or stylus.

A non-exhaustive list of the sensors considered for a smart pen or digital writing instrument is provided below:
Force and/or pressure sensors are sensors, along with strain gauges when placed inside the writing instrument which allow for measuring the force applied between the pen's tip and the writing surface. Handwriting and sketching forces have been considered of diagnostic value for patients with mild cognitive impairment.

Accelerometers, gyroscopes, or their combination, i.e., in the form of Inertial Measurement Units (IMU) are used for tremor analysis as well as other parameters for patient classification. These types of sensors enable the determination of the writing speed, which is considered as a metric for identifying bradykinesia or other hypokinetic hand movement symptoms, like dyskinesia, dystonia and motor fluctuations.

Magnetometers can be used in a smart pen to measure the writing angle between the writing instrument and the writing plane or surface. Tracking of the pen's orientation and/or direction enables the assessment of the user's hand rigidity and tremor.

Optical sensors can be used in a smart pen to record the handwriting output, or the evolution of handwriting operation can be done by considering optical sensors mounted directly on the smart pen. Abnormalities in writing style, such as micrographia, can be identified using these sensors and have been reported to correlate with PD.

Time of Flight (ToF) proximity sensors can be involved in the tracking the inter-foot distance during walking. This setup can be reconfigured to track the hovering status of the pen during writing. Longer on-air times may indicate deterioration in a person's cognitive skills.

Regarding writing action, handwriting is the writing action done with a writing instrument, such as a pen or pencil, using the hand. Handwriting includes both printing and cursive styles. Handwriting involves the use of a pen or pencil which the user keeps holding with his hand/fingers even when he does not write. At a writing session the user writes or sketches with a writing instrument. A writing session includes actual handwriting instances and on-air instances in which a tip of the writing instrument is not touching a writing surface.

Because each person's handwriting is unique and different, it can be used to verify a document's writer. Significant changes or the deterioration of a person's handwriting may be a symptom or a result of several different neurological diseases.

Certain terms are used in the following manner in the present disclosure:
The expression "digital device" may refer to an electronic device that uses discrete, numerable data and processes for all its operations, and is capable of displaying content to the user. Examples of such a device include but are not limited to: Mobile phones, Laptops, Tablets, Personal computers, Netbooks, iPads, etc.

The expression "haptic or tactile feedback" may refer to a physical response on a digital device from the user input, also known as kinaesthetic communication. The application with mechanical or electronic means of force, vibrations, or motions to the user in response to a user's action on the digital device.

The expression "human machine interface" may refer to a user interface or dashboard or an input system that connects a person to a machine, system, or device. For example, a physical button on a remote controller.

The expression "group of users" may refer to a panel of users selected through specific methodology, with the scope to test products and provide feedback, in order to provide statistical insights to specific parameters of the products and their respective use.

The expression "connected to" is not limited to a direct connection between two objects (i.e., it is not required that the two objects abut). For instance, a first object and a second object can be indirectly connected through a third object (e.g., a cutting edge portion can be indirectly connected to an edge of the base portion through another component such as a blade mounting portion).

### Description of the Drawings

**Fig. 1** illustrates an overview of components of a writing instrument for monitoring handwriting and/or sketching changes of its user according to the present disclosure.
**Fig. 2** illustrates a process flow diagram of a writing instrument according to the present disclosure.
**Fig. 3** illustrates a process flow diagram of a writing instrument according to the present disclosure.
**Fig. 4** illustrates a perspective view of a writing instrument according to the present disclosure.
**Fig. 5** illustrates a method flow chart for monitoring handwriting and/or sketching changes of a writing instrument's user according to the present disclosure.

### Detailed Description

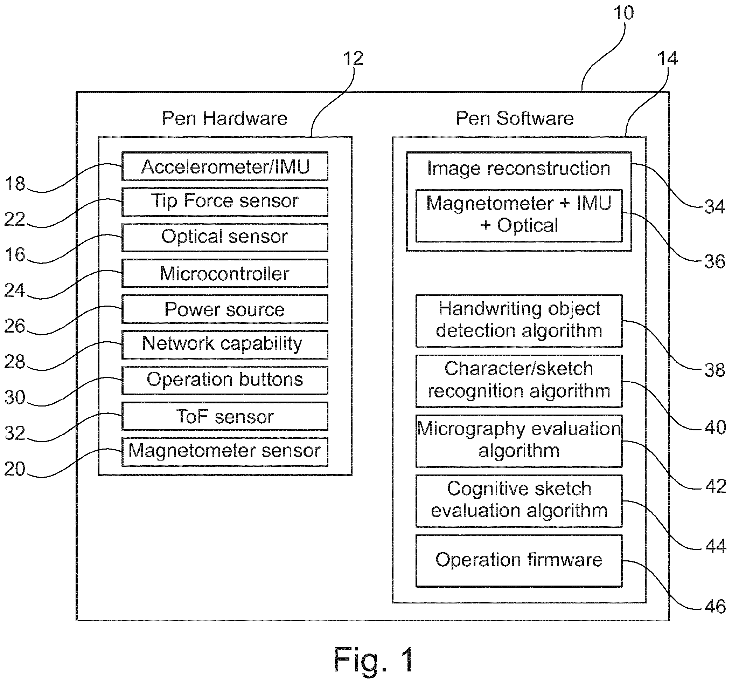

**Fig. 1** shows an overview of components of a writing instrument 10 for monitoring and/or identifying handwriting and/or sketching changes of its user according to the present disclosure. The writing instrument 10 can be named smart pen, digital writing instrument or digital device. The writing instrument 10 includes the pen hardware 12 and the pen software 14.

In embodiments, the writing instrument 10 or system could be named handwriting changes recognition system, capable of identifying handwriting and sketching changes of a user during handwriting and evaluating if there is any abnormality. The handwriting changes recognition system includes or consists of two subsystems i.e., a handwriting detection smart pen system or pen hardware 12 and a handwriting changes evaluation software or pen software 14.

The pen hardware 12 is capable of detecting character and sketching changes like size and spacing changes, sketch inconsistencies that may relate to neurodegenerative diseases with sensors incorporated on a smart pen. The sensors may be one, more or all of the following: an IMU, a magnetometer, a force/pressure sensor, a ToF sensor and an optical sensor.

As shown in **Fig. 1****,** the pen hardware 12 of the writing instrument 10 includes one or more optical sensors 16 and/or cameras with macro lenses which may be mounted on the main body of the writing instrument to provide sequential visual data of the handwriting/sketching operation for a more rapid assessment and identification of abnormalities.

The optical sensor may be placed at different locations on the body of the writing instrument depending on the types of the other sensing elements considered.

In embodiments, the optical sensor may be placed near the pen's tip and be synchronized with the contact sensor (force/pressure/strain gauge or ToF proximity sensor) to capture frames of each individual stroke. The output of each stroke is then synthesized in a digital representation of the writing/sketching output based on the orientation and location data provided by a magnetometer or IMU or other sensors.

In embodiments, the optical sensor may be placed on the top end of the writing instrument for capturing a wider writing area. In this case the outer geometry of the writing instrument has to be properly configured to allow for the user to grip the writing instrument in such away so that his/her hand doesn't block the writing/sketching output.

In embodiments, the technical specifications for the optical sensor may include at least one of a resolution of 1280x720 pixels for photos and 1920x1080 pixels or higher for video, a weight not larger than 50 gr., a voltage of 5V DC, a framerate of 30 fps to 200 fps and size smaller than 7.0x5.5x4.0 mm.

In embodiments, the optical sensor system may also include an Optic Image Stabilization (OIS) mechanism and/or a digital image stabilization software (EIS).

The pen hardware 12 further includes an accelerometer /IMU sensor 18 and a magnetometer sensor 20.

At least one accelerometer, gyroscope or combination in the form of an IMU and at least one magnetometer placed either on the same location as the IMU or at a different position inside the writing instrument are used to capture the position, the orientation, the speed and the acceleration of the writing instrument 10 for the digital reconstructing of the handwriting and/or sketching trajectory.

The accelerometer, gyroscope and magnetometer sensors considered in the embodiments of the system are providing output for all the 3-axis of motion and are capable of capturing 6 DoF (Degrees of Freedom), i.e., three translational and three rotational directions.

In embodiments, the bandwidth for the accelerometer may be selected to be between 20 to 100 Hz.

In embodiments, the accuracy as well as the precision of the magnetometer sensor may not be below 0,2 mm to capture accurately the position of the pen in space, while the orientation output should be less than 0,5°.

In embodiments, the size may not exceed 5,0 x 5,0 x 5,0 mm.

The writing instrument 10 may include a tip force sensor 22 or paper contact sensor.

One or more force and/or pressure sensor and/or strain gauge is placed inside the body of the writing instrument, either aligned with the longitudinal axis of the pen or at an inclined configuration, to record the developed force between the nib the writing surface during the handwriting operation.

In embodiments, a force/pressure sensor may have at least 0,01 N resolution, measure up to 10 N of force and be capable of identifying 4086 pressure levels or more.

In embodiments, its size may be no more than 2,5 x 2,5 x 1,0 mm in order to fit inside the writing instrument and be placed along its longitudinal axis or at an inclined configuration without affecting the ergonomics of the writing instrument.

In embodiments, this component when combined with the orientation measurement given by the magnetometer determines the components of the force acting parallel and perpendicular to the writing plane. This sensor may act both as an activator for enabling other functions as well as for recording the magnitude of the actual writing force applied by the user of the writing surface.

The pen hardware 12 of the writing instrument 10 may further include a microcontroller 24.

A microcontroller (MCU) may process and control all the sensors, circuits and functions of the smart pen and may be of conventional wearable type or may be able to perform advanced AI processing. It may contain a flash memory module.

Microcontroller may be a conventional ultra-low power MCU suitable for wearable applications such as but not limited, a 16 or 32-bit-ARM MCU. In examples, the microcontroller may be based on a platform such as customizable single-chip ASIC AI or be based on a RISC-V AI architecture. The microcontroller may have a custom operating firmware. The memory unit allows both for temporary storage of generated data until transfer to the logging device for offline processing as well as for online processing.

The pen hardware 12 of the writing instrument 10 further includes a power source 26. The power source 26 can power the electronic components of the smart pen and can include one or more disposable batteries, plug-in rechargeable batteries, and/or a wireless inductive charging module. The power source and the associated electronic circuit may be capable of delivering up to 12 V and exemplary 3 V.

The pen hardware 12 of the writing instrument 10 further includes network capability 28 especially wireless network capability.

Wireless connectivity enables the smart pen to interact with other devices. The network capability 28 may support at least one of the different wireless protocols such as Wi-Fi, ANT+, Bluetooth Low Energy (BLE), IEEE 802.15.4.

The pen hardware 12 of the writing instrument 10 further includes operation buttons 30.

The control or operation buttons 30 can be located on the sides or on the periphery of the smart pen and their type can be one of the following
- Touch buttons
- Switches
- Rotation switches
- Sliding buttons

In an embodiment of the smart pen physical buttons may be omitted and the smart pen may have its operation controlled by gestures, voice commands and/or is remotely controlled via another device such as PC, notebook, tablet, smartphone, smartwatch etc.

The pen hardware 12 of the writing instrument 10 further includes a Time-of-Flight sensor 32 which may be an alternative to the tip force sensor 22 or paper contact sensor.

The Time-of-Flight sensor 32 or proximity sensor can be placed on the outer shell of the writing instrument, used to track the hovering status of the pen during writing. One or more time-of-flight sensors 32 can be incorporated in the smart pen or writing instrument 10 either single or multi zone, with a field of view not less than 60° and a range up to 250 mm.

The Time-of-Flight (ToF) sensor is used for measuring the distance between the pen and the writing surface, based on the time difference between the emission of a signal and its return to the sensor, after being reflected by the writing surface. Various types of signals (also called carriers) can be used with the Time-of-Flight principle, the most common being sound and light.

The pen hardware 12 of the writing instrument 10 may further include a grip force sensor. One or more flexible pressure sensing pads may be wrapped around a tubular-shape of a main pen body, used to capture the distribution of the gripping forces applied by the user.

The flexible pressure sensitive pads can be either force resistive sensors that allow detection of squeezing or force sensitive resistive matrix sensor consisting of an array of force sensing cells arranged in rows and columns. This configuration allows for measuring both the magnitude of the force applied as well as the location, resulting in a force distribution output mapping the gripping action.

In embodiments, the size of the sensor matrix does not exceed 50 x 75 mm.

In embodiments, the measured force resolution may range between 75 g and 2,5 kg.

In embodiments, a resolution of 1%, a device rise time above 10 kHz and a lifecycle of more than a million activations is considered for the operation.

The writing instrument 10 may further include a human machine interface (HMI) output like for example a notification LED.

One or more notification LED may be placed on the pen's body. These may be used to notify the user of the different operations performed by the pen, it's operational status, battery life, memory status, data transfer as well as suggest him/her to seek medical assistance.

In embodiments, the notification may be achieved by different colors of the LED and/or by different flash patterns.

In embodiments, the visual notification may be accompanied by audio or haptic feedback signals.

In embodiments, the at least one LED light source is considered for the following purposes:
- as an element of interaction with the user to indicate the operational status of the device (on/off),
- writing operation (contact of the pen with the writing surface),
- battery health and/or status,
- memory storage status,
- handwriting disorder notification.

The operation or execution of the methods and/or algorithms may be on the writing instrument 10 or another operating device like e.g., a tablet, a smartphone, a smartwatch, a PC or any other suitable device capable of running the operation and visualisation application. The operating device is capable of wireless communication with the smart pen sending and receiving data. For example, the wireless communication may be achieved through Bluetooth or Wi-fi connection.

In embodiments, the operating device may include, one or all the required algorithms and software for the processing and calculation of the data of the smart pen.

In embodiments, the operating device may include the user interface with which the user may interact with the smart pen and visualise the measurement results.

In embodiments, the operating device may alert the user in case of abnormal measurement data.

The pen software 14 of the writing instrument 10 can also be phrased as handwriting changes evaluation software capable of calculating and evaluating letter and sketching parameter changes related to neurodegenerative diseases and alerting user whether they are normal or abnormal. One, some or all of the handwriting changes evaluation software elements may be embedded in the smart writing instrument 10 or in the accompanying operating device.

The handwriting changes evaluation software or pen software 14 may include or consist of the following elements.

The pen software 14 of the writing instrument 10 includes an image reconstruction algorithm 34 that takes as input the magnetometer, IMU and optical sensor data 36 and reconstructs the image of the handwriting action (letters or sketched lines).

This involves pre-processing handwriting positional data, either in the form of coordinate timeseries or bitmap images, or a combination of both.

With respect to the combination of magnetometer/IMU data, the first methodological part relates to the fusion of the data from the two sensors using a data processing algorithm like a Kalman filter. This improves the accuracy of the results. The data sampling frequency is at minimum 200 Hz and at maximum 1000 Hz. The coordinate data, along with the pen tip force information are combined to classify each discrete timestep into writing/non writing classes. The digital representation of the handwritten/sketching session is reconstructed using Bezier interpolation on the writing timesteps' coordinate data. To further improve the accuracy of the results, the magnetometer data are further combined with the optical sensor data. The same digital presentation as described above can be extracted by joining the local images from the optical sensor using as a guideline the global positional and orientation information from the magnetometer. In this case, high accuracy of the positional data is not required since they are only used for preliminary alignment of optical images. The two images are then superimposed and aggregated to acquire the final result.

The pen software 14 of the writing instrument 10 further includes handwriting and sketching object detection algorithms 38 and 40 that take as an input the reconstructed images of the handwritten text/sketch and detects the letter parameters of interest (size, spacing, identification).

The object detection process may be performed using a suitable deep Neural Network. The architecture of the network can be one of many established ones, such as Centernet, Resnet or R-CNN. The training starts using pretrained networks on the Coco dataset and are finetuned for identifying characters/other appropriate objects.

The output of the model for each document is a set of bounding boxes (and their coordinates) for each identified object, as well as the classification of that object, e.g., one letter was identified at position (xmin, ymin) = (0,1, 0,3), (xmax, ymax) = (0,15, 0,4) and was classified with best probability 0,9 as being letter 'd'. Using the above information, we can compute and compare the size characteristics of the same letters. The height of each letter is taken to be ymax - ymin, the width xmax-xmin, the center of gravity as ((xmax-xmin)/2, (ymax-ymin)/2) and finally the area as the product of height multiplied by the width.

The pen software 14 of the writing instrument 10 further includes a micrography evaluation algorithm 42 that compares the current letter size and spacing properties of the user to the historical letter size and spacing properties of the same user and outputs the differences.

In case of significant reduction of letter size or spacing, it notifies user of abnormal results.

For example, OCR can be employed to segment and recognize each letter. For each identified letter, the size is quantified via the height and width of its bounding box, while the distance between two consecutive letters is computed as the minimum distance between their respective bounding boxes.

The timeseries of consecutive letter distances, and these of sizes (height, width, area) of the same letters are then analyzed via timeseries decomposition to determine if there is a progressive deterioration (trend) in size/inter letter distance

The pen software 14 of the writing instrument 10 further includes a cognitive sketch evaluation algorithm 44 that compares the current sketch properties of the user to a historical or database benchmark sketch and outputs the differences.

In case of significant differences or inconsistencies between the current and benchmark sketch, it notifies user of abnormal results.

For example, in case of sketching of the clock (Cog test), the following processing is required:
A first step is the algorithm 44 to perform a check that all 12 numbers were written, and if the order of writing is correct. Subsequently, the centers of the numbers' bounding boxes are joined by straight lines and the resulting shape is compared to a dodecagon using the Hausdorff distance, to establish how well the shape of the clock was drawn.

Furthermore, the algorithm 44 checks the existence of the two clock arrows and extracts their length (diagonal of the bounding box) and direction (via distance of the arrowhead to the closest bounding box corner). This allows to verify whether the user drew the correct time.

The same applies to other types of cognitive testing sketches with the relevant change in properties of interest depending on the case.

The pen software 14 of the writing instrument 10 may further include operation and visualization software or operation firmware 46.

This is a software that takes the results of the system algorithms and visualises them to the user in order to alert him of normal or abnormal handwriting changes.

It also permits the user to set specific parameters of the measurement sensors and communicate them to the smart pen.

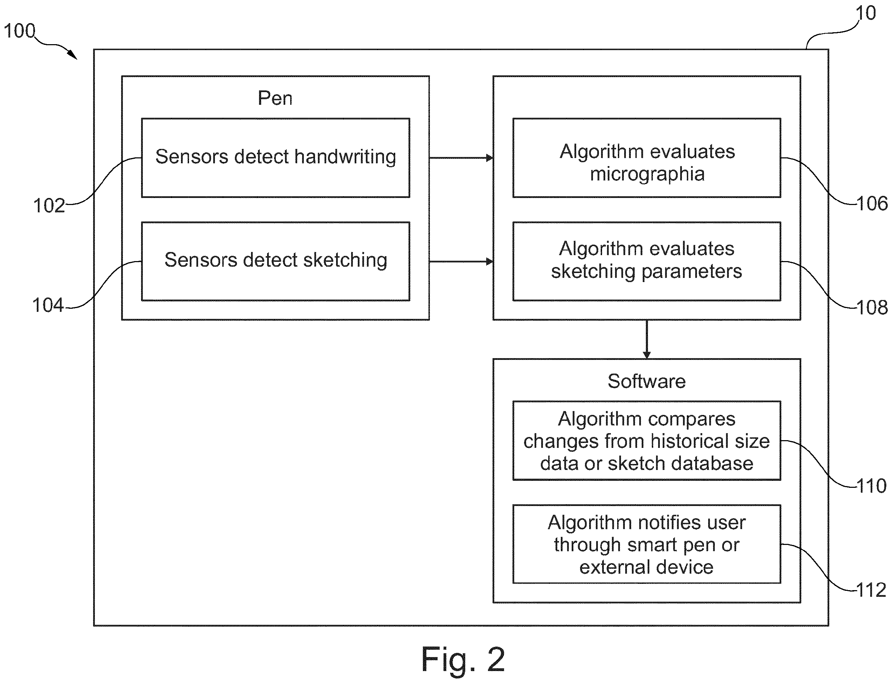

**Fig. 2** illustrates a process flow diagram 100 of a writing instrument 10 according to the present disclosure.

In a first step 102, sensors detect handwriting using for example the handwriting object detecting algorithm 38. In step 104, the sensors detect sketching using for example the character/sketch recognition algorithm 40.

The output of step 102 is inputted into step 106 in which the micrography evaluation algorithm 42 evaluates signs of micrographia. The output of step 104 is input to step 108 in which the cognitive sketch evaluation algorithm 44 evaluates sketching power meters.

The outputs of steps 106 and 108 are input to step 110 where an algorithm compares changes from the current writing and/or sketching to previous writing and/or sketching in the same writing session, to historical size data and/or to a sketch database.

At step 112, and algorithm notifies the user through the writing instrument 10 or an external device in case of an abnormal result of the comparing.

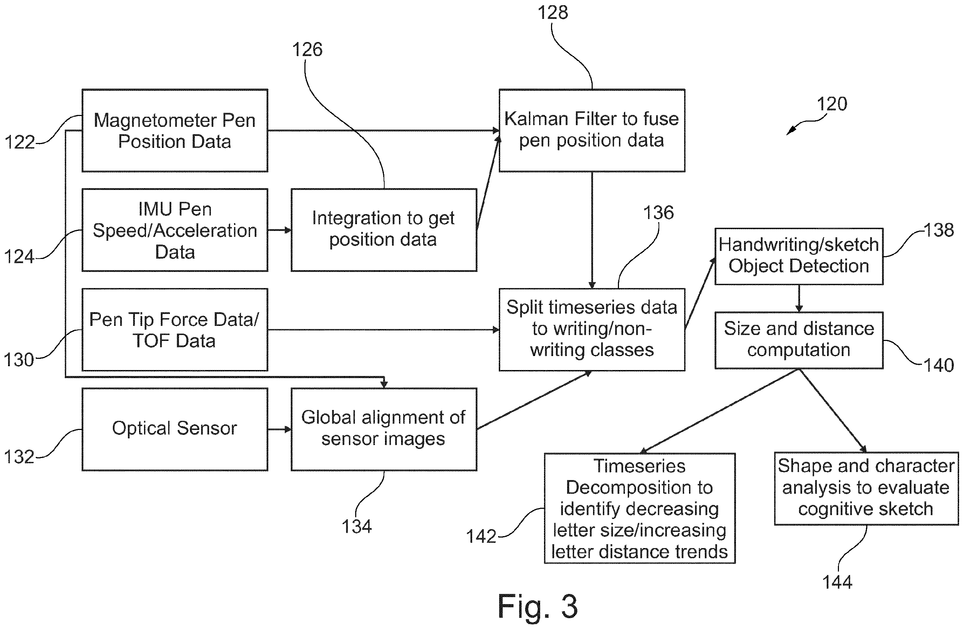

**Fig. 3** illustrates a process flow diagram 120 of a writing instrument 10 according to the present disclosure.

The steps according to the process flow diagram 120 describe the correlation of functions of the pen software 14 as depicted in Fig. 1. Here, the steps according to the process flow diagram 120 are referenced towards the functions of the pen software 14.

The image reconstruction algorithm 34 that takes as input the magnetometer, IMU and optical sensor data 36 includes the magnetometer pen position data 122, the IMU pen speed/acceleration data 124, the pen tip force data/TOF data 130, the optical sensor 132, the integration to get position data 126, the global alignment of sensor images 134, the Kalman filter to fused pen position data 128, and the split time series data to writing/non-writing classes 136. The steps 132 and 134 may be omitted..

The positional data 122 and 124 are fused by the Kalman filter 128 and split into timeseries of writing/non-writing data in step 136 using the input of airtime of step 130. The term writing/non-writing also includes sketching/non-sketching.

The handwriting and sketching object detection algorithms 38 and 40 include the steps handwriting/sketching object detection 138 and size and distance computation 140.

The micrography evaluation algorithm 42 includes the step timeseries decomposition to identify decreasing letter size/increasing letter distance trends 142 according to which the presence or an indication of micrography is identified.

The shape and correct the analysis to evaluate cognitive sketch 144 includes the cognitive sketch evaluation algorithm 44 that compares the current sketch properties of the user to a historical or database benchmark sketch and outputs the differences.

In an example of sketching of a clock (Cog test), the first step may include to check the recognized clock numbers. The second step may include to check whether centers of bounding boxes are connected and compare them to an ideal dodecagon. And a third step may include to compute directions of the clock arrows and to evaluate the correctness of the time.

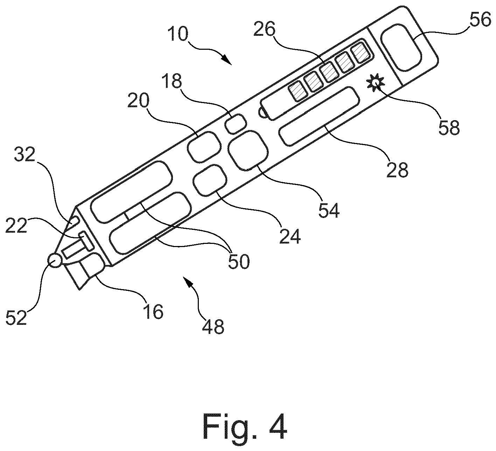

**Fig. 4** illustrates a perspective view of a writing instrument 10 according to the present disclosure. The writing instrument 10 can correspond to the writing instrument 10 as depicted in **Fig. 1** and can include or execute the algorithm shown in Figs. 2 and 3.

The writing instrument 10 includes network capability 28 for communicating with an external device. The writing instrument 10 further includes sensors like the tip force sensor 22, the grip force sensor 48 including flexible pressure sensing pads 50, the magnetometer 20, the IMU 18, the time-of-flight sensor 32 and additionally an optical sensor 16. The optical sensor 16 is arranged close to a tip 52 of the writing instrument 10 and can be used to capture handwriting details to support the algorithm. The power source 26 supplies the writing instrument 10 and its components with power.

The microcontroller 24 is connected with the sensors and adapted to execute the algorithm shown in Figs. 2 and 3. The memory unit 54 is connected to the microcontroller 24 and adapted to store raw data and computed data. An USB port 56 is further connected to the microcontroller 24 for external communication. An LED 58 can indicate the status of the writing instrument 10 and/or an indication to the user that the handwriting and/or sketching changes which were monitored and/or identified during the writing sessions are abnormal.

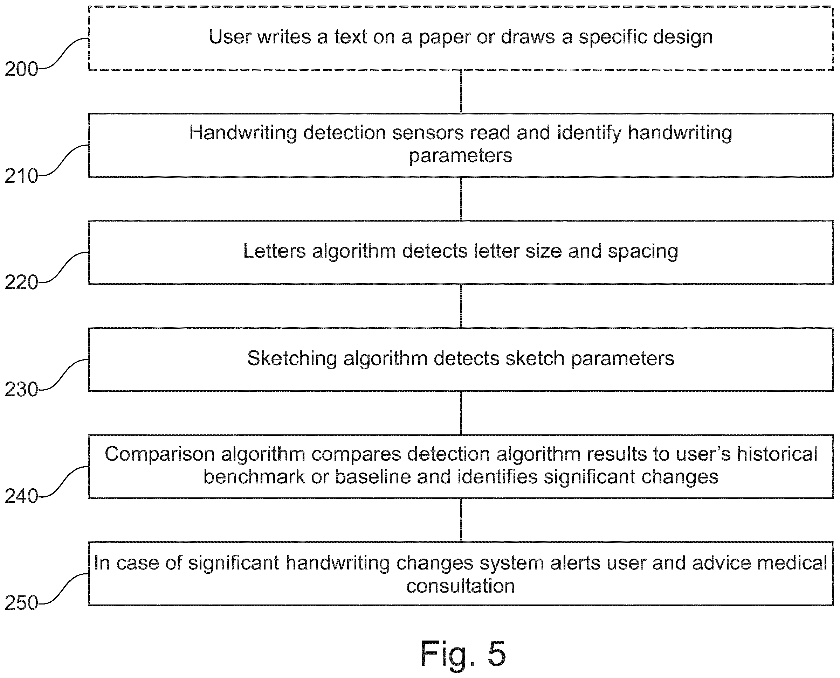

**Fig. 5** illustrates a method flow chart for monitoring and/or identifying handwriting and/or sketching changes of a writing instrument's user according to the present disclosure.

In a first step 200 of the method, a user writes a text on a paper or draws a specific design. This may include that the user takes smart pen to perform handwriting or sketching activities on a writing surface.

In a second step 210, handwriting detection sensors read and identify handwriting parameters. This may include that smart pen sensors detect and digitize letters, words, or sketches.

In other words, the second step 210 includes monitoring and/or identifying a handwriting and/or sketching trajectory and motion parameters of the user during a writing and/or sketching session with the writing instrument by reading data from the sensors of the writing instrument.

The monitoring and/or identifying handwriting and/or sketching changes may include monitoring and/or identifying at least one of a writing force between the writing instrument's tip and a writing surface, motion and distance information including position, orientation, speed, and/or acceleration of the writing instrument, sequential visual data of the handwriting/sketching operation output, and a force distribution and/or gripping strength applied by the user's fingers on the writing instrument.

The monitoring and/or identifying handwriting and/or sketching changes may include computing a time span of the writing instrument not in contact with the writing surface between two consecutive writing strokes from the monitored force.

In a third step 220, a letter algorithm detects or calculates letter size and distribution or spacing.

In other words, the third step 220 includes evaluating letter and word segmentation and identification data obtained from the monitoring and further includes quantifying size and distance of letters of the evaluated data.

In a fourth step 230, a sketching algorithm calculates sketching parameters.

In other words, the fourth step 230 includes quantifying size and distance of sketch components and parameters of the evaluated data.

In examples, storing monitored data and/or quantified data as historical data may be included as an additional step.

In a fifth step 240, a comparison algorithm compares detection algorithm results to user's historical benchmark or baseline and identifies significant changes. This may include an algorithm based on letter sizing and distribution parameters which evaluates micrographia by comparing user's current status with historical data as well as writing session's statistics (average letter size, average character distance). This may also include an algorithm based on sketching parameters which evaluates sketching inconsistencies by comparing current sketches to a database of normal cognitive sketches.

In other words, the fifth step 240 includes comparing the quantified data to at least one of data from the actual writing and/or sketching session, historical data from the same user, and reference data from a group of users.

In a sixth step 250, in case of significant handwriting changes the system alerts the user and advice medical consultation. Thus, an algorithm may alert the user whether handwriting and/or sketches are normal or abnormal.

In other words, the sixth step 250 includes providing an indication in case of an abnormal result of the comparing.

The steps as depicted in Figs. 2 and 3 can be included in the method of Fig. 5.

The present disclosure also relates to the computer-implemented method for monitoring and/or identifying handwriting and/or sketching changes of a writing instrument's user and the writing instrument for monitoring and/or identifying handwriting and/or sketching changes of its user of the following aspects:
1. A computer-implemented method for monitoring handwriting and/or sketching changes of a writing instrument's user, comprising:
   - monitoring a handwriting and/or sketching trajectory and motion parameters of the user during a writing and/or sketching session with the writing instrument by reading data from the sensors of the writing instrument;
   - evaluating letter and word segmentation and identification data obtained from the monitoring;
   - quantifying size and distance of letters and/or sketch components and parameters of the evaluated data;
   - storing monitored data and/or quantified data as historical data;
   - comparing the quantified data to at least one of data from the actual writing and/or sketching session, historical data from the same user, and reference data from a group of users; and
   - providing an indication in case of an abnormal result of the comparing.
2. The method of aspect 1, wherein monitoring handwriting and/or sketching changes includes monitoring and/or identifying at least one of a writing force between the writing instrument's tip and a writing surface, motion and distance information including position, orientation, speed, and/or acceleration of the writing instrument, sequential visual data of the handwriting/sketching operation output, and a force distribution and/or gripping strength applied by the user's fingers on the writing instrument.
3. The method of aspect 2, wherein monitoring handwriting and/or sketching changes includes:
   - computing a time span of the writing instrument not in contact with the writing surface between two consecutive writing strokes from the monitored force.
4. The method of one of the preceding aspects, wherein writing instrument motion data is derived from a magnetometer sensor and an inertial measurement unit, IMU, sensor of the writing instrument.
5. The method of aspect 4, comprising:
   - fusing writing instrument motion data through a data processing algorithm Kalman filter, wherein the writing instrument motion data are multidimensional, wherein the dimensions are at least one of position, orientation, speed, and acceleration.
6. The method of aspect 5, comprising:
   - sorting the fused writing instrument motion data into writing and non-writing parts by using data of a writing force between the writing instrument's tip and a writing surface.
7. The method of aspect 6, the sorting comprising:
   - digitizing handwriting input of the writing session; and
   - correlating the digitized handwriting input with motion data of the writing instrument.
8. The method of aspect 6 or 7, wherein evaluating includes
   - identifying at least one of size, spacing, and identification parameters of characters and/or shapes of the writing parts.
9. The method of aspect 8, wherein comparing includes
   - comparing the current characters' and/or shapes' size and spacing parameters of the user to the historical characters and/or shapes size and spacing parameters of the same user and/or
   - comparing current sketch properties of the user to a historical sketch and/or a reference sketch.
10. The method of aspect 8, comprising:
   - performing a check that all twelve numbers of a clock of a Cog test are written and that the order of writing is correct;
   - providing straight lines between centers of the numbers' bounding boxes and comparing the resulting shape to a dodecagon;
   - checking the existence of the two clock arrows; and
   - extracting the length of the two clock arrows as the diagonal of the bounding box and the direction via distance of the arrowhead to the closest bounding box corner.
11. A writing instrument for monitoring handwriting and/or sketching changes of its user, comprising:
   - one or more sensors configured to monitor a handwriting and/or sketching trajectory and motion parameters of the user during a writing and/or sketching session with the writing instrument by reading data from the sensors of the writing instrument;
   - a computer system configured to execute the computer-implemented method for monitoring handwriting and/or sketching changes of a writing instrument's user according to one of the preceding aspects;
   - a storage system configured to store monitored data and/or quantified data as historical data; and
   - a communication system configured to provide an indication based on the computer-implemented method.
12. The writing instrument of aspect 11, comprising at least one of:
   - a hand presence sensor configured to obtain a force distribution and/or a gripping strength applied by the user's fingers on the writing instrument;
   - a force tip sensor configured to obtain a writing force between the writing instrument's tip and a writing surface;
   - at least one motion sensor configured to obtain at least one of position, orientation, speed, and acceleration of the writing instrument;
   - a sensor, such as an optical sensor, configured to provide sequential visual data of the handwriting/sketching operation; and
   - a proximity sensor configured to obtain a hovering status distance of the writing instrument's tip above the writing surface.
13. The writing instrument of aspect 12, wherein the proximity sensor is a time-of-flight sensor, wherein the hand presence sensor comprises a plurality of flexible pressure sensing pads, wherein the force tip sensor comprises a force and/or pressure sensor configured to measure a force and/or pressure of a tip of the writing instrument, wherein the optical sensor comprises an optic image stabilization mechanism and/or a digital image stabilization algorithm, and/or wherein the at least one motion sensor comprises at least one of a magnetometer, a gyroscope, an accelerometer, and an inertial measurement unit, IMU.
14. The writing instrument of one of aspects 11 to 13, comprising:
   - a digitizing system configured to digitize content of a writing and/or sketching session.
15. The writing instrument of one of aspects 11 to 14, the writing instrument comprising:
   - a user interface configured to receive input from a user and/or to provide information to the user such as the indication; and/or
   - a wireless communication system configured to provide the indication via an external device having an interface.

## Claims

1. A computer-implemented method for monitoring handwriting and/or sketching changes of a writing instrument's user, comprising:
- monitoring a handwriting and/or sketching trajectory and motion parameters of the user during a writing and/or sketching session with the writing instrument (10) by reading data from the sensors of the writing instrument (10);
- evaluating letter and word segmentation and identification data obtained from the monitoring;
- quantifying size and distance of letters and/or sketch components and parameters of the evaluated data;
- storing monitored data and/or quantified data as historical data;
- comparing the quantified data to at least one of data from the actual writing and/or sketching session, historical data from the same user, and reference data from a group of users; and
- providing an indication in case of an abnormal result of the comparing.

2. The method of the preceding claim, wherein monitoring handwriting and/or sketching changes includes monitoring and/or identifying at least one of a writing force between the writing instrument's tip (52) and a writing surface, motion and distance information including position, orientation, speed, and/or acceleration of the writing instrument (10), sequential visual data of the handwriting/sketching operation output, and a force distribution and/or gripping strength applied by the user's fingers on the writing instrument (10).

3. The method of claim 2, wherein monitoring handwriting and/or sketching changes includes:
- computing a time span of the writing instrument (10) not in contact with the writing surface between two consecutive writing strokes from the monitored force.

4. The method of any one of the preceding claims, wherein writing instrument (10) motion data is derived from a magnetometer sensor (20) and an inertial measurement unit sensor (18) of the writing instrument (10).

5. The method of claim 4, comprising:
- fusing writing instrument motion data through a data processing algorithm, wherein the writing instrument (10) motion data are multidimensional, wherein the dimensions are at least one of position, orientation, speed, and acceleration.

6. The method of claim 5, comprising:
- sorting the fused writing instrument (10) motion data into writing and non-writing parts by using data of a writing force between the writing instrument's tip (52) and a writing surface.

7. The method of claim 6, the sorting comprising:
- digitizing handwriting input of the writing session; and
- correlating the digitized handwriting input with motion data of the writing instrument (10).

8. The method of claim 6 or 7, wherein evaluating includes
- identifying at least one of size, spacing, and identification parameters of characters and/or shapes of the writing parts.

9. The method of claim 8, wherein comparing includes
- comparing the current characters' and/or shapes' size and spacing parameters of the user to the historical characters and/or shapes size and spacing parameters of the same user and/or
- comparing current sketch properties of the user to a historical sketch and/or a reference sketch.

10. The method of claim 8, comprising:
- performing a check that all twelve numbers of a clock of a Cog test are written and that the order of writing is correct;
- providing straight lines between centers of the numbers' bounding boxes and comparing the resulting shape to a dodecagon;
- checking the existence of the two clock arrows; and
- extracting the length of the two clock arrows as the diagonal of the bounding box and the direction via distance of the arrow head to the closest bounding box corner.

11. A writing instrument (10) for monitoring handwriting and/or sketching changes of its user, comprising:
- one or more sensors configured to monitor a handwriting and/or sketching trajectory and motion parameters of the user during a writing and/or sketching session with the writing instrument (10) by reading data from the sensors of the writing instrument (10);
- a computer system (24) configured to execute the computer-implemented method for monitoring handwriting and/or sketching changes of a writing instrument's user according to one of the preceding claims;
- a storage system configured to store monitored data and/or quantified data as historical data; and
- a communication system configured to provide an indication based on the computer-implemented method.

12. The writing instrument (10) of claim 11, comprising at least one of:
- a hand presence sensor configured to obtain a force distribution and/or a gripping strength applied by the user's fingers on the writing instrument (10);
- a force tip sensor (22) configured to obtain a writing force between the writing instrument's tip (52) and a writing surface;
- at least one motion sensor (18, 20) configured to obtain at least one of position, orientation, speed, and acceleration of the writing instrument (10);
- a sensor (16), such as an optical sensor, configured to provide sequential visual data of the handwriting/sketching operation; and
- a proximity sensor (32) configured to obtain a hovering status of the writing instrument's tip (52) above the writing surface.

13. The writing instrument (10) of claim 12, wherein the proximity sensor (32) is a time-of-flight sensor, wherein the hand presence sensor comprises a plurality of flexible pressure sensing pads (50), wherein the force tip sensor (22) comprises a force and/or pressure sensor configured to measure a force and/or pressure of a tip (52) of the writing instrument (10), wherein the optical sensor (16) comprises an optic image stabilization mechanism and/or a digital image stabilization algorithm, and/or wherein the at least one motion sensor (18, 20) comprises at least one of a magnetometer (20), a gyroscope, an accelerometer, and an inertial measurement unit (18).

14. The writing instrument (10) of any one of claims 11 to 13, comprising:
- a digitizing system configured to digitize content of a writing and/or sketching session.

15. The writing instrument (10) of any one of claims 11 to 14, the writing instrument (10) comprising:
- a user interface configured to receive input from a user and/or to provide information to the user such as the indication; and/or
- a wireless communication system configured to provide the indication via an external device having an interface.
